# EUROPEAN PATENT SPECIFICATION

(11) **EP 3 558 188 B1**
(45) Date of publication and mention of the grant of the patent: **21.01.2026**
(21) Application number: 17884472.6
(22) Date of filing: 22.12.2017
(51) Int. Cl.: A61F 13/02, A61F 13/00, A61F 15/00

(54) **ACTIVATED CARBON COMPOSITE WOUND DRESSING**
ZUSAMMENGESETZTER WUNDVERBAND MIT AKTIVKOHLE
PANSEMENT COMPOSITE AU CHARBON ACTIF

(30) Priority: 23.12.2016 US 201662438651 P
(43) Date of publication of application: 30.10.2019
(73) Proprietor: Calgon Carbon Corporation, Moon Township, PA 15108 (US)
(72) Inventor: LAVOCAH, Wayne, South Shields Durham NE34 7NX (GB); CURTIS, Paul, Murton Durham SR7 9GQ (GB); BROWN, Robert, Preston Lancashire PR4 6TG (GB); SLEIGH, Dean, Leeds Yorkshire LS25 3AS (GB)
(74) Representative: Müller-Boré & Partner Patentanwälte PartG mbB
(86) International application number: PCT/US2017/068193
(87) International publication number: WO 2018/119398

(56) References cited:
- GB-A- 2 206 495
- GB-A- 2 531 345
- GB-A- 2 531 345
- US-A- 4 789 699
- US-A1- 2007 135 785
- US-B1- 6 303 700

## Description

### BACKGROUND ART

GB 2 531 345 A describes a composite wound dressing comprising a superabsorbent material, and a layer of activate carbon cloth.

### SUMMARY

The present invention is as set out in the appended claims 1 to 19.

### BRIEF DESCRIPTION OF THE DRAWINGS

For a fuller understanding of the nature and advantages of the present invention, reference is made to the following detailed description taken in connection with the accompanying drawings, in which:
FIG. 1 is a schema illustrating a composite wound dressing.
FIG. 2 is a graph illustrating the free swell capacity and retention capacity after loading of a number of cloths. The data labeled "Zorflex Plus" is a composite wound dressing in accordance with the present invention.

### DETAILED DESCRIPTION

Before the present compositions and methods are described, it is to be understood that they are not limited to the particular compositions, methodologies, or protocols described, as these may vary. It is also to be understood that the terminology used in the description is for the purpose of describing the particular versions only, and is not intended to limit their scope, which will be limited only by the appended claims.

It must also be noted that as used herein and in the appended claims, the singular forms "a," "an," and "the" include plural references unless the context clearly dictates otherwise. Unless defined otherwise, all technical and scientific terms used herein have the same meanings as commonly understood by one of ordinary skill in the art. Although any methods and materials similar or equivalent to those described herein can be used in the practice or testing disclosed, the preferred methods, devices, and materials are now described.

"Optional" or "optionally" means that the subsequently described event or circumstance may or may not occur, and that the description includes instances where the event occurs and instances where it does not.

As used herein, the term "about" means plus or minus 10% of the numerical value of the number with which it is being used. Therefore, about 50mm means in the range of 45mm to 55mm.

The present invention is directed to a composite wound dressing including an activated carbon material and a method for preparing such a composite wound dressing. Herein, methods for treating wounds by applying the composite wound dressing to a wound are further described.

According to the present invention, the composite wound dressing includes a wound-interfacing layer including an activated carbon material. "Wound interfacing" as described herein shall mean that the activated carbon material of the wound-interfacing layer directly contacts, covers or otherwise faces the wound, and substantially no other layer of the composite wound dressing contacts, covers or otherwise directly faces the wound. The activated carbon material may include woven cloth, non-woven cloth, knitted cloth, activated carbon felt, or combinations thereof. The activated carbon material may include an activated carbon cloth. A suitable activated carbon cloth includes activated carbon cloth sold under the trade name Zorflex^{®} (FM30K) and is commercially available from Calgon Carbon.

The activated carbon material may include activated carbon particles, activated carbon powder, activated carbon fiber, or a combination of these materials. For example, activated carbon particles may be immobilized or attached to a non-activated carbon-based cloth and alternatively, activated carbon particles, powder, and/or fibers may be contained between sealed layers of a non-activated carbon based cloth. Alternatively, activated carbon particles, powder, and/or fibers may be included in a woven, non-woven, knitted, or felt activated carbon cloth material.

The activated carbon material may include borax, i.e., sodium borate, sodium tetraborate, or disodium tetraborate. Without wishing to be bound by theory, the presence of borax in the activated carbon material may enhance the anti-microbial activity of the activated carbon in the absence of a known anti-microbial agent. Any amount of borax may be included in the activated carbon material. For example, the amount of borax within the activated carbon material may be about 0.001 wt. % to 50 wt. %, may be about 0.01 wt. % to 30 wt. %, or may be about 0.1 wt. % to 25 wt. %.

As discussed above, anti-microbial activity can be achieved in the absence of other known anti-microbial agents, because activated carbon inherently has antimicrobial activity, and this activity can be enhanced by the addition of borax. However, the activated carbon material may further include one or more anti-microbial agents other than activated carbon. For example, the activated carbon material may include a noble metal such as silver, gold, palladium, platinum, copper, zinc, or a combination thereof, and preferably the noble metal may be silver or zinc. An amount of the noble metal within the activated carbon material may be about 0.001 wt. % to about 30 wt. % or about 0.01 wt. % to about 10 wt. %.

The noble metal may be provided as noble metal particles or powder, wherein the particle size of the noble metal particles may be less than about 100 nm, less than 50 nm, and less than 25 nm. Noble metal particles or powder can be associated with the activated carbon in the activated carbon material, any means known in the art can be used to create such an association including, but not limited to, thermocracking, electroplating, electroless plating, or vacuum plating. Noble metal particles or powder may be associated with activated carbon material, such as an activated carbon cloth, by immersion. For example, activated carbon fibers or cloth may be immersed in a solution of silver nitrate for 1 to 720 minutes at a pH of 3 to 8, which reduces the silver allowing the silver to form particles on the surface of the activated carbon fiber or cloth. Such methods may further include drying the fiber or cloth to remove residual water, which is typically carried out at a temperature of from about 25°C to about 150°C. Silver-carrying activated carbon fiber prepared according to the above process generally results in activated carbon fibers having a BET surface area of greater than about 400 m²/g, carbon content of greater than about 50 wt. %, silver content of greater than about 0.001 wt. %, and a density of greater than about 1.8 g/m³.

A therapeutically active agent can be included in the activated carbon material. The therapeutically active agent provided may be pre-adsorbed into the pore volume of the material and its subsequent release from the material may be controlled. The release of the therapeutically active agent may be controlled by applying an electrical current to the material. Examples of suitable therapeutically active agents may include antibiotics, antimicrobials, sulfonamides, antiseptics, analgesics, or anesthetics and other medicaments and substances used to promote healing such as osmotic colloids, protease inhibitors, proteolytic enzymes, growth factors, steroidal or non-steroidal anti-inflammatory drugs, nutrients, and antioxidants, and any combination of therapeutically active agents. Examples of particular active agents that may be useful may include, but are not limited to, acrisorcin, haloprogin, iodochlorhydroxyquin, tolnaftate, triacetin, centella asiatica, econazole nitrate, mafenide, mupirocin, povidone iodine, chlohexidine, silver sulfadiazine, povidone iodine, silver salts, triclosan, sucralfate, quaternary ammonium salts, tetracycline, penicillins, terramycins, erythromycin, bacitracin, neomycin, polymycin B, mupirocin, clindamycin, and any mixtures thereof.

The activated carbon material may further include one or more non-toxic, pharmaceutically, and dermatologically acceptable carriers, diluents, or excipients or combinations thereof. The therapeutically active agent and/or carriers, diluents, and/or excipients may be prepared for topical use and can be in various dosage forms including, but not limited to, a gel, a paste, an ointment, a cream, an emulsion, or a suspension. For example, a suitable thickener such as, aluminum stearate or hydrogenated lanolin, or gelling agent can be added to an aqueous or oil base to formulate an ointment or cream. Examples of suitable excipients include starch, tragacanth, cellulose derivative, polyethylene glycol, silicones, bentonite, silicic acid, talc, or a mixture thereof. The activated carbon can be mixed with the therapeutically active agent, carrier, diluent, and/or excipient and other active components to provide the desired dosage form.

The composite wound dressing of the present invention further includes a barrier material. The barrier material layer provides a barrier between the wound-interfacing layer including the activated carbon material and a liquid-absorbing material layer, described below. According to the present invention, the barrier material layer is configured to permit the passage of exudate leaking from a wound to the liquid-absorbing layer, while preventing the exudate from leaking back into the activated carbon material. The barrier material layer may disperse the exudate and confine the exudate into the liquid-absorbing material layer. The barrier material layer may include a polymer, such as a hydrophilic polymer. Examples of suitable polymers include, but are not limited to, polyethylene, polypropylene, polyester, polyamides such as nylons, fluoropolymers such as polyvinylidene fluoride (PVDF) or polytetrafluoroethylene (PTFE), ethylene methyl acrylate (EMA), and mixtures thereof. The polymers may be drug-impermeable. Such drug impermeable materials include, for example, polyvinyl chloride, polyvinyl dichloride, polyurea, polyolefins, such, but not limited to, ethylene vinylacetate copolymer, polyethylene, and polypropylene, and polyesters, such as, but not limited to, polyethylene terephthlate, and combinations and mixtures of these. The barrier material layer may include a non-woven fabric.

The barrier material layer may be directly applied over the wound-interfacing layer on the wound-opposing side of the wound-interfacing layer. Alternatively, the barrier material layer may be applied to the wound-interfacing layer with a first adhesive. An exemplary first adhesive suitable for adhering the barrier material layer to the wound-interfacing layer includes a nonwoven web of a thermoplastic adhesive polymer resin. Other first adhesives are further contemplated, and such first adhesives would be apparent to one of skill in the art in view of this disclosure. Such other suitable first adhesives may include any adhesive or combination of adhesives known in the medical arts. In general, the first adhesive may be moisture vapor transmitting and pressure-sensitive, meaning that it forms a bond when applied with light pressure, of the type conventionally used for island-type wound dressings. First adhesives include, but are not limited to, acrylate ester copolymers, polyvinyl ethyl ether and polyurethane pressure sensitive adhesives. The relative thickness of the first adhesive layer may vary and may be optimized based on the type of adhesive used. For example, the basis weight of the first adhesive layer may be from about 20 g/m² to about 250 g/m² or from about 50 g/m² to 150 g/m². The first adhesive may be a polyurethane-based pressure sensitive adhesive.

According to the present invention, the composite wound dressing includes a liquid-absorbing material layer. As contemplated herein, the liquid-absorbing material is able to hold a significantly high volume of liquid, i.e., wound exudate, without leaking. For example, the liquid-absorbing material layer may have an absorbency of about 4.645 to about 185.805 grams of liquid per square meter (about 50 to about 2,000 grams of liquid per square foot) of material. The absorbency of the liquid-absorbing material layer may be from about 9.290 to about 139.353 grams of liquid per square meter (about 100 to about 1,500 grams per square foot). The liquid-absorbing material layer may comprise a sodium polyacrylate polymer dispersed between layers of cellulose paper. The liquid-absorbing material layer may have a weight of about 80 to about 300 grams per square meter. The configuration of the liquid-absorbing material layer in the composite wound dressing reduces gel leakage, and in some cases reduces gel leakage under pressure, when compared to commercially available wound dressings. The liquid-absorbing material layer may comprise a liquid-absorbing pad.

The composite wound dressing of the present invention further includes a liquid-impermeable material outer layer. The liquid-impermeable material may be breathable and fluid repellant. In other words, the liquid-impermeable material may allow for the transfer of gases or air to the wound while preventing liquid or exudate from leaking through the liquid-impermeable material. According to the present invention, the liquid-impermeable material includes polyethylene. The liquid-impermeable material layer may be a polyethylene spunbond fabric. The liquid-impermeable material may be semi-permeable. For example, the liquid-impermeable material may be permeable to water vapor but not permeable to liquid. Suitable materials for liquid-impermeable material may have a moisture vapor transmission rate (MVTR) of, for example, about 300 g/m²/24 hrs. to about 5000 g/m²/24 hrs., or from about 500 g/m²/24 hrs. to about 2000 g/m²/24 hrs. at about 37°C at 100% to 10% relative humidity. The liquid-impermeable material may have a thickness of from about 10 µm to about 1000 µm or from about 100 µm to about 500 µm.

A second adhesive may be included to adhere the liquid-absorbing material layer to the liquid impermeable material outer layer. This second adhesive may include a polyvinylpyrrolidone/vinyl acetate copolymer. Other possible second adhesives may be employed and are discussed herein.

According to the present invention, a bond is formed to bond at least a portion of the barrier material layer and the liquid-impermeable material outer layer to the wound-interfacing layer. The bond may include a circumferential seal between the barrier material layer and the liquid-impermeable outer layer with the wound-interfacing layer where the bond encompasses a substantially sealed pouch of un-bonded layers of the composite. In this context, the liquid-absorbing material layer is positioned in the pouch and is not bonded to the circumferential seal. "Circumferential" as used herein with reference to the bond or seal shall mean a bond or seal having a boundary in a continuous circuit. The bond or seal may be a thermal weld. The bond can be in any shape as desired, and the bond can take the desired shape of a finally formed composite wound dressing. Suitable shapes include, without limitations, a square, a rectangular, a round, a butterflied, or another desired shape.

The composite wound dressings can be used to treat any kind of wound such as, for example, lacerations, cuts, scrapes, abrasions, post-operative wounds, denuded skin, and burns, or other skin problems (e.g., allergies) and dressings of various sizes can be prepared such that minor wounds as well as larger wounds can be treated using the composite wound dressings. In general, the composite dressing may allow transfer of air and moisture vapor into and out of the wound and trapping or capturing wound exudate while being liquid-impermeable and immobilizing microbes on the activated carbon. The composite wound dressings described herein can be used to treat wounds on humans or any other animal including, but not limited to, mammals, fish, reptiles, birds, and other creatures. Thus, medical and veterinary uses for the composite wound dressings described herein are encompassed, and such uses can be carried out by trained medical professionals, physicians, veterinarians, nurses, and emergency medical technicians, or by consumers who purchase the wound dressings described herein over the counter.

The composite wound dressing may be directly applied to a wound, such as those described above. Therefore, herein described is a composite wound dressing which can be applied to a wound, and the composite wound dressing may be shaped to adequately cover a wound. Such various shapes are described herein. The composite wound dressing designed and configured to be applied to a wound may include one or more additional components that are provided to, for example, improve healing, reduce adhesion to the wound, improve adherence to skin surrounding the wound, reduce itching, or otherwise aid in improving patient comfort. Additional components may include, without limitation, topical compositions including one or more therapeutic agents. Any type of topical composition including, but not limited to, gels, pastes, ointments, creams, emulsions, or suspensions containing the therapeutic agent may be applied.

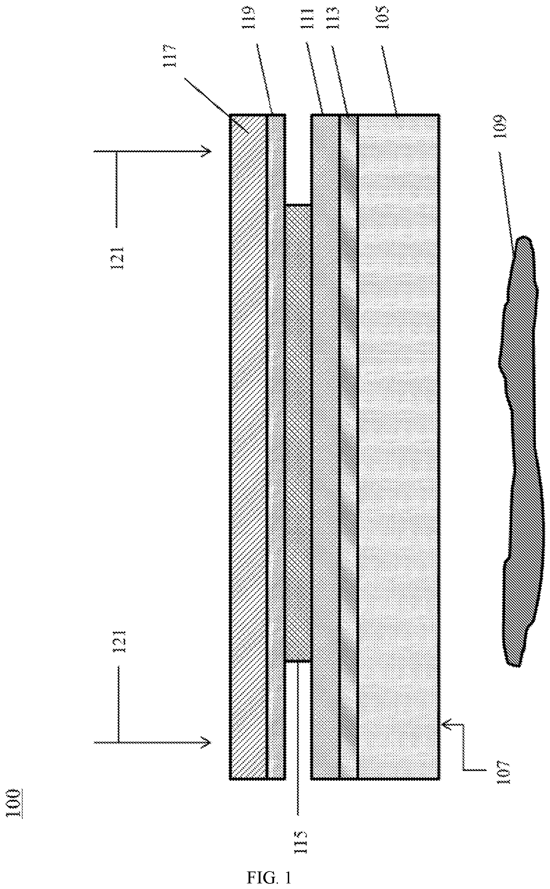

FIG. 1 illustrates an exemplary composite wound dressing **100.** The composite wound dressing includes a first layer **105** having an activated carbon material. The activated carbon material includes activated carbon cloth sold under the trade name Zorflex^{®} (FM30K). The first layer includes a wound interface **107** that directly interfaces, contacts, covers, or otherwise faces a wound **109.** The composite **100** further includes a barrier layer **111** that is adhered to the first layer **105** by a first adhesive layer **113.** The first adhesive layer **113** may be applied to either the wound opposing side of the first layer **105** or the wound facing side of the barrier layer **111.** Once the first adhesive layer **113** is applied, pressure, or other suitable action, may be used to aid in securing the barrier layer **111** to the first layer **105.**

A liquid-absorbing pad **115** is provided on a wound-opposing side of the barrier layer **111.** The barrier layer **111** allows the passage of liquid exudate from the wound **109** to the liquid-absorbing pad **115,** but prevents exudate captured by the liquid absorbing pad **115** from leaking back into the first layer **105** and/or back into the wound **109.** The barrier layer **111** includes a hydrophilic non-woven material. The liquid-absorbing pad **115** includes a superabsorbent pad having a weight of about 80 to about 300 grams per square meter. In the exemplary composite wound dressing **100** of FIG. 1, the liquid absorbing pad **115** is a superabsorbent sodium polyacrylate polymer which is commercially available under the trade name Gelok^{®}. The liquid-absorbing pad **115** may be positioned in the composite wound dressing **100** while having a length shorter than the other layers within the composite wound dressing **100.** This can allow the liquid-absorbing pad **115** to be entirely encapsulated in the composite wound dressing **100** after forming a seal or bond, as described below.

The outer layer **117** of the composite wound dressing **100** is a liquid-impermeable material that prevents liquid from entering the composite wound dressing **100** and prevents exudate capture by the liquid-absorbing pad **115** from leaking out of the composite wound dressing **100.** The liquid-absorbing material includes a breathable material that can allow the passage of air and/or moisture vapor across the liquid-impermeable material while preventing the passage of liquids. The liquid-impermeable material includes a polyethylene sheet or membrane. In the exemplary composite wound dressing **100** the outer layer **117** is adhered to the liquid-absorbing layer **115** by, optionally, a second adhesive layer **119.** The optional second adhesive layer **119** can be applied to either the wound opposing side of the liquid-absorbing layer **115** or the wound-facing side of the outer layer **117.** Once the second adhesive layer **119** is applied, pressure, or other suitable action, may be used to aid in securing the liquid-absorbing layer **115** to the outer layer **117.** The second adhesive layer **119** includes a polyvinylpyrrolidone/vinyl acetate copolymer adhesive.

Once the layers are formed into a composite, the composite wound dressing **100** can be sealed to create a circumferential sealed bond or bead that may resemble the final shape of the composite wound dressing **100.** A thermal heat weld may be used to create the sealed bead. In this context, a thermal weld head can be applied to contact and seal the composite wound dressing **100** along arrows **121.** Although FIG. 1 illustrates a two-dimensional view of the wound dressing composite, it is contemplated that the thermal weld head can be in a circumferential shape of the finally produced composite wound dressing. Suitable shapes are disclosed herein. The thermal weld head provides heat which melts and seals the outer layer **117,** the barrier layer **111** and the first and second adhesive layers **113, 119** to the first layer **105,** thereby creating a bond to the activated carbon material of the first layer **105.** The bond comprises a narrow sealing interface resulting in a sealed outer edge surrounding an unsealed pocket of the layers of the composite wound dressing **100.** Thus, the layers of the composite wound dressing **100** within the areas sealed by the bond are unsealed and are encapsulated by the sealed bond. In the exemplary composite wound dressing **100** of FIG. 1, the liquid-absorbing layer **115** is not sealed upon contact with the thermal weld held, but, rather, remains encapsulated in the unsealed pocket. Once the composite wound dressing**100** is sealed by the thermal weld head, the excess unsealed material beyond the outer periphery of the bond can be trimmed to result in a finally formed composite wound dressing **100.**

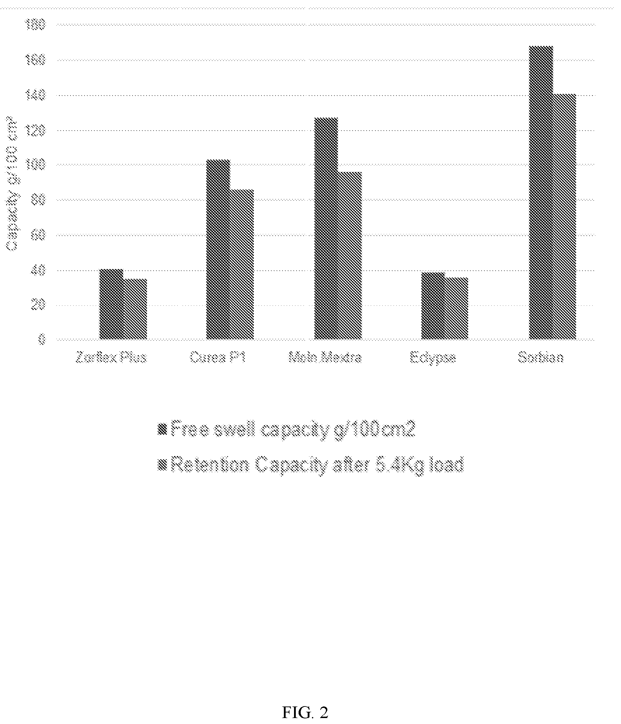

FIG. 2 illustrates the free swell capacity (in g/100cm²) and retention capacity after a 5.4kg load for a number of cloths, including Zorflex Plus, Curea P1, Moln.Mextra, Eclypse, and Sorbian. The cloth labeled "Zorflex Plus" is a composite wound dressing in accordance with the present invention.

Generally, the composite wound dressings described above may be sterile and can be packaged in a microorganism-impermeable container such as a pouch. Further, the composite wound dressing of the present invention is provided against the leakage of liquid exudate from a wound and/or against gel leaking from the liquid-absorbing material under pressure conditions. The composite wound dressing is lightweight and flexible.

A preferable method for producing the composite wound dressing of the present invention includes providing a wound-interfacing layer including an activated carbon material. A barrier material is applied to the wound-opposing side of the wound-interfacing layer. The method includes applying a first adhesive to secure the barrier material to the wound-interfacing layer. A liquid-absorbing material layer is applied to the wound opposing side of the barrier material. The liquid-absorbing material layer may include a super absorbent pad as described herein. A liquid-impermeable material outer layer including a liquid-impermeable material fabric comprising polyethylene is applied to the wound-opposing side of the liquid-absorbing material layer. A second adhesive is applied to secure the liquid-absorbing material layer to the liquid-impermeable material layer. At least a portion of the outer layer and the barrier material layer and, optionally, the first and second adhesive materials, are bonded to the wound-interfacing layer forming a sealed pouch of un-bonded layers of the composite. The barrier material layer is configured to permit the passage of exudate leaking from a wound to the liquid-absorbing layer, while preventing the exudate from leaking back into the activated carbon material. The bonding of the layers of the composite may be accomplished by a thermal weld. The bonding may include forming a circumferential seal encompassing a sealed pouch of un-bonded layers of the composite. The sealed pouch may include the liquid-absorbing layer. The method may further include trimming excess layers outside periphery of the sealed pouch, resulting in a finally formed composite wound dressing. Herein described are suitable materials for each layer of the composite.

Herein further described are methods for using the composite wound dressings described above. The methods generally include the step of contacting a wound with the composite wound dressing.

The composite wound dressing described herein can be used to treat any type of wound, including lacerations, cuts, scrapes, abrasion, post-operative wounds, denuded skin, and burns. The composite wound dressing can remain in contact with the wound throughout the healing process. The composite wound dressings disclosed herein provide a dressing that can handle excessive amounts of exudation from a wound. Thus, wounds that are known to generate excessive amounts of exudate would benefit from the composite wound dressings due to the wound dressings ability to absorb the exudate, wick and keep the exudate away from the wound site. The anti-microbial activity of the activated carbon material in the wound-interfacing layer may also reduce the likelihood of infection by trapping and eradicating microbes in the wound at the time the composite wound dressing is applied and throughout the healing process by immobilizing microbes before they can reach the wound itself.

## Claims

1. A composite wound dressing (100) comprising:
a wound-interfacing layer (105) including an activated carbon material;
a barrier material layer (111) adjacent to a wound-opposing side of the wound-interfacing layer (105);
a liquid-absorbing material layer (115) adjacent to a wound-opposing side of the barrier material layer (111);
a liquid-impermeable outer layer (117) adjacent to a wound-opposing side of the liquid-absorbing material layer (115) and comprising polyethylene; and
a bond substantially bonding a portion of the liquid-impermeable outer layer (117) to the wound-interfacing layer (105), the bond forming a sealed pouch of un-bonded layers of the composite wound dressing (100) and encapsulating the liquid-absorbing material layer (115),
wherein the barrier material layer (111) is configured to permit the passage of exudate leaking from a wound to the liquid-absorbing material layer (115),
while preventing the exudate from leaking back into the activated carbon material.

2. The composite wound dressing (100) of claim 1, wherein the activated carbon material comprises:
a. an activated carbon cloth;
b. a woven cloth, non-woven cloth, knitted cloth, activated carbon felt, or combinations thereof; or
c. activated carbon particles, activated carbon powder, activated carbon fiber, or combinations thereof.

3. The composite wound dressing (100) of claim 1, wherein the barrier material layer (111) comprises a non-woven fabric.

4. The composite wound dressing (100) of claim 1, wherein the liquid-absorbing material layer (115) has an absorbency of about 75 to about 1,700 grams per square foot.

5. The composite wound dressing (100) of claim 1, wherein the liquid-impermeable outer layer comprises a polyethylene spunbond fabric.

6. The composite wound dressing (100) of claim 1, further comprising a first adhesive (113) disposed between the wound-interfacing layer (105) and the barrier material layer (111).

7. The composite wound dressing (100) of claim 6, wherein the first adhesive (113) comprises a nonwoven web of a thermoplastic polymer resin.

8. The composite wound dressing (100) of claim 1, further comprising a second adhesive (119) disposed between the liquid-absorbing material layer (115) and the liquid impermeable outer layer (117).

9. The composite wound dressing (100) of claim 8, wherein the second adhesive (119) comprises a polyvinylpyrrolidone/vinyl acetate copolymer.

10. The composite wound dressing (100) of claim 1, wherein the bond comprises a thermal weld.

11. The composite would dressing (100) of claim 1, wherein the bond comprises a circumferential seal encompassing the sealed pouch.

12. A method for producing a composite wound dressing (100) in accordance with any one of claims 1-11, the method comprising:
providing a wound-interfacing layer (105) including an activated carbon material;
applying a barrier material layer (111) to a wound-opposing side of the wound-interfacing layer (105);
applying a liquid-absorbing material layer (115) to a wound-opposing side of the barrier material layer (111);
applying a liquid-impermeable outer layer (117) including a liquid-impermeable material fabric comprising polyethylene to a wound-opposing side of the liquid-absorbing material layer (115); and
bonding at least a portion of the liquid-impermeable outer layer (117) and the barrier material layer (111) to the wound-interfacing layer (105) to form a sealed pouch of un-bonded layers of the composite wound dressing (100) and encapsulating the liquid-absorbing material layer (115),
wherein the barrier material layer (111) is configured to permit the passage of exudate leaking from a wound to the liquid-absorbing material layer (115),
while preventing the exudate from leaking back into the activated carbon material.

13. The method of claim 12, wherein applying the barrier material layer (111) to the wound-interfacing layer (105) further comprises applying a first adhesive (113).

14. The method of claim 13, wherein the first adhesive (113) comprises a nonwoven web of thermoplastic polymer resin.

15. The method of claim 14, wherein applying the liquid-impermeable outer layer (117) to the liquid-absorbing material layer (115) comprises applying a second adhesive (119).

16. The method of claim 15, wherein the second adhesive (119) comprises a polyvinylpyrrolidone/vinyl acetate copolymer.

17. The method of claim 16, wherein the bonding comprises forming a thermal weld.

18. The method of claim 17, wherein the bonding comprises forming a circumferential seal encompassing the sealed pouch.

19. The composite wound dressing (100) as recited in claim 1 wherein the barrier material layer (111) is adhered to the wound-interfacing layer (105) with a first adhesive (113); and
the liquid-impermeable outer layer (117) is adhered to the liquid absorbing material layer (115) with a second adhesive (119).

## Patentansprüche

1. Komposit-Wundverband (100), umfassend:
eine Wundzwischenschicht (105), die ein Aktivkohlematerial einschließt;
eine Barrierematerialschicht (111) angrenzend an eine der Wunde zugewandte Seite der Wundzwischenschicht (105);
eine flüssigkeitsabsorbierende Materialschicht (115), die an eine der Wunde zugewandte Seite der Barrierematerialschicht (111) angrenzt;
eine flüssigkeitsundurchlässige Außenschicht (117), die an eine der Wunde zugewandte Seite der flüssigkeitsabsorbierenden Materialschicht (115) angrenzt und Polyethylen umfasst; und
eine Verbindung, die einen Teil der flüssigkeitsundurchlässigen Außenschicht (117) im Wesentlichen mit der Wundzwischenschicht (105) verbindet, wobei die Verbindung einen versiegelten Beutel aus unverbundenen Schichten des Komposit-Wundverbands (100) bildet und die Schicht aus flüssigkeitsabsorbierendem Material (115) einkapselt,
wobei die Barrierematerialschicht (111) so konfiguriert ist, dass sie den Durchgang von Exsudat, das aus einer Wunde austritt, zur flüssigkeitsabsorbierenden Materialschicht (115) ermöglicht, während sie gleichzeitig verhindert, dass das Exsudat zurück in das Aktivkohlematerial austritt.

2. Komposit-Wundverband (100) nach Anspruch 1, wobei das Aktivkohlematerial umfasst:
a. eine Aktivkohle-Tuch;
b. einen gewebten Stoff, einen Vliesstoff, einen gestrickten Stoff, ein Aktivkohlefilz oder Kombinationen davon; oder
c. Aktivkohleteilchen, Aktivkohlepulver, Aktivkohlefasern oder Kombinationen davon.

3. Komposit-Wundverband (100) nach Anspruch 1, wobei die Barrierematerialschicht (111) ein Vlies umfasst.

4. Komposit-Wundverband (100) nach Anspruch 1, wobei die flüssigkeitsabsorbierende Materialschicht (115) eine Absorptionsfähigkeit von etwa 75 bis etwa 1.700 Gramm pro Quadratfuß aufweist.

5. Komposit-Wundverband (100) nach Anspruch 1, wobei die flüssigkeitsundurchlässige Außenschicht ein Polyethylen-Spinnvlies umfasst.

6. Komposit-Wundverband (100) nach Anspruch 1, der weiter einen ersten Klebstoff (113) umfasst, der zwischen der Wundzwischenschicht (105) und der Barrierematerialschicht (111) angeordnet ist.

7. Komposit-Wundverband (100) nach Anspruch 6, wobei der erste Klebstoff (113) ein Vlies aus einem thermoplastischen Polymer umfasst Harz.

8. Komposit-Wundverband (100) nach Anspruch 1, der weiter einen zweiten Klebstoff (119) umfasst, der zwischen der flüssigkeitsabsorbierenden Materialschicht (115) und der flüssigkeitsundurchlässigen Außenschicht (117) angeordnet ist .

9. Komposit-Wundverband (100) nach Anspruch 8, wobei der zweite Klebstoff (119) ein Polyvinylpyrrolidon/Vinylacetat-Copolymer umfasst.

10. Komposit-Wundverband (100) nach Anspruch 1, wobei die Verbindung eine thermische Schweißung umfasst.

11. Komposit-Wundverband (100) nach Anspruch 1, wobei die Verbindung eine den versiegelten Beutel umgebende Umfangsversiegelung umfasst.

12. Verfahren zur Herstellung eines Komposit-Wundverbands (100) gemäß einem der Ansprüche 1-11, wobei das Verfahren umfasst:
Bereitstellen einer Wundzwischenschicht (105), die ein Aktivkohlematerial einschließt;
Aufbringen einer Barrierematerialschicht (111) auf eine der Wunde zugewandte Seite der Wundzwischenschicht (105);
Aufbringen einer flüssigkeitsabsorbierenden Materialschicht (115) auf eine der Wunde zugewandte Seite der Barrierematerialschicht (111);
Aufbringen einer flüssigkeitsundurchlässigen Außenschicht (117), die ein flüssigkeitsundurchlässiges Materialgewebe aus Polyethylen umfasst, auf eine der Wunde zugewandte Seite der flüssigkeitsabsorbierenden Materialschicht (115); und
Verbinden mindestens eines Teils der flüssigkeitsundurchlässigen Außenschicht (117) und der Barrierematerialschicht (111) mit der Wundzwischenschicht (105), um einen versiegelten Beutel aus unverbundenen Schichten des Komposit-Wundverbands (100) zu bilden und
die flüssigkeitsabsorbierende Materialschicht (115) einzukapseln,
wobei die Barrierematerialschicht (111) so konfiguriert ist, dass sie den Durchgang von aus einer Wunde austretendem Exsudat zur flüssigkeitsabsorbierenden Materialschicht (115) ermöglicht, gleichzeitig aber verhindert, dass das Exsudat zurück in das Aktivkohlematerial austritt.

13. Verfahren nach Anspruch 12, wobei das Aufbringen der Barrierematerialschicht (111) auf die Wundzwischenschicht (105) weiter das Aufbringen eines ersten Klebstoffs (113) umfasst.

14. Verfahren nach Anspruch 13, wobei der erste Klebstoff (113) ein Vlies aus thermoplastischem Polymerharz umfasst.

15. Verfahren nach Anspruch 14, wobei das Aufbringen der flüssigkeitsundurchlässigen Außenschicht (117) auf die flüssigkeitsabsorbierende Materialschicht (115) das Aufbringen eines zweiten Klebstoffs (119) umfasst.

16. Verfahren nach Anspruch 15, wobei der zweite Klebstoff (119) ein Polyvinylpyrrolidon/Vinylacetat-Copolymer umfasst.

17. Verfahren nach Anspruch 16, wobei das Verbinden das Bilden einer thermischen Schweißnaht umfasst.

18. Verfahren nach Anspruch 17, wobei das Verbinden das Bilden einer den versiegelten Beutel umfassenden Umfangsversiegelung umfasst.

19. Komposit-Wundverband (100) nach Anspruch 1, wobei die Barrierematerialschicht (111) mit einem ersten Klebstoff (113) an der Wundzwischenschicht (105) haftet; und
die flüssigkeitsundurchlässige Außenschicht (117) mit einem zweiten Klebstoff (119) an der flüssigkeitsabsorbierenden Materialschicht (115) befestigt ist.

## Revendications

1. Pansement composite (100) comprenant :
une couche d'interface avec la plaie (105) incluant un matériau de charbon actif ;
une couche de matériau barrière (111) adjacente à un côté opposé à la plaie de la couche d'interface avec la plaie (105) ;
une couche de matériau absorbeur de liquide (115) adjacente à un côté opposé à la plaie de la couche de matériau barrière (111) ;
une couche externe imperméable au liquide (117) adjacente à un côté opposé à la plaie de la couche de matériau absorbeur de liquide (115) et comprenant du polyéthylène ; et
un lien reliant essentiellement une portion de la couche externe imperméable au liquide (117) à la couche d'interface avec la plaie (104), le lien formant une poche scellée de couches non liées du pansement composite (100) et encapsulant la couche de matériau absorbeur de liquide (115),
dans lequel la couche de matériau barrière (111) est configurée pour permettre le passage d'exsudat fuyant d'une plaie vers la couche de matériau absorbeur de liquide (115) tout en empêchant l'exsudat de fuir en retour dans le matériau de charbon actif.

2. Pansement composite (100) selon la revendication 1, dans lequel le matériau de charbon actif comprend :
a. un tissu de charbon actif ;
b. un tissu tissé, tissu non tissé, tissu tricoté, feutre de charbon actif ou des combinaisons de ceux-ci ; ou
c. des particules de charbon actif, de la poudre de charbon actif, de la fibre de charbon actif ou des combinaisons de celles-ci.

3. Pansement composite (100) selon la revendication 1, dans lequel la couche de matériau barrière (111) comprend un tissu non tissé.

4. Pansement composite (100) selon la revendication 1, dans lequel la couche de matériau absorbeur de liquide (115) a une absorbance d'environ 75 à environ 1700 grammes par pied carré.

5. Pansement composite (100) selon la revendication 1, dans lequel la couche externe imperméable au liquide comprend un tissu filé-lié de polyéthylène.

6. Pansement composite (100) selon la revendication 1, comprenant en outre un premier adhésif (113) disposé entre la couche d'interface avec la plaie (105) et la couche de matériau barrière (111).

7. Pansement composite (100) selon la revendication 6, dans lequel le premier adhésif (113) comprend une toile non tissée d'une résine polymère thermoplastique.

8. Pansement composite (100) selon la revendication 1, comprenant en outre un second adhésif (119) disposé entre la couche de matériau absorbeur de liquide (115) et la couche externe imperméable au liquide (117).

9. Pansement composite (100) selon la revendication 8, dans lequel le second adhésif (119) comprend un copolymère de polyvinylpyrrolidone/acétate de vinyle.

10. Pansement composite (100) selon la revendication 1, dans lequel le lien comprend une soudure thermique.

11. Pansement composite (100) selon la revendication 1, dans lequel le lien comprend un joint circonférentiel englobant la poche scellée.

12. Procédé de production d'un pansement composite (100) selon l'une quelconque des revendications 1 à 11, le procédé comprenant :
fournir une couche d'interface avec la plaie (105) incluant un matériau de charbon actif ;
appliquer une couche de matériau barrière (111) à un côté opposé à la plaie de la couche d'interface avec la plaie (105) ;
appliquer une couche de matériau absorbeur de liquide (115) à un côté opposé à la plaie de la couche de matériau barrière (111) ;
appliquer une couche externe imperméable au liquide (117) incluant un tissu en matériau imperméable au liquide comprenant du polyéthylène à un côté opposé à la plaie de la couche de matériau absorbeur de liquide (115) ; et
relier au moins une portion de la couche externe imperméable au liquide (117) et
la couche de matériau barrière (111) à la couche d'interface avec la plaie (105) pour former une poche scellée de couches non liées du pansement composite (100) et encapsuler la couche de matériau absorbeur de liquide (115),
dans lequel la couche de matériau barrière (111) est configurée pour permettre le passage d'exsudat fuyant d'une plaie vers la couche de matériau absorbeur de liquide (115) tout en empêchant l'exsudat de fuir en retour dans le matériau de charbon actif.

13. Procédé selon la revendication 12, dans lequel appliquer la couche de matériau barrière (111) à la couche d'interface avec la plaie (105) comprend en outre appliquer un premier adhésif (113).

14. Procédé selon la revendication 13, dans lequel le premier adhésif (113) comprend une toile non tissée d'une résine polymère thermoplastique.

15. Procédé selon la revendication 14, dans lequel appliquer la couche externe imperméable au liquide (117) à la couche de matériau absorbeur de liquide (115) comprend appliquer un second adhésif (119).

16. Procédé selon la revendication 15, dans lequel le second adhésif (119) comprend un copolymère de polyvinylpyrrolidone/acétate de vinyle.

17. Procédé selon la revendication 16, dans lequel le lien comprend former une soudure thermique.

18. Procédé selon la revendication 17, dans lequel le lien comprend former un joint circonférentiel englobant la poche scellée.

19. Pansement composite (100) selon la revendication 1, dans lequel la couche de matériau barrière (111) est collée à la couche d'interface avec la plaie (105) avec un premier adhésif (113) ; et
la couche externe imperméable au liquide (117) est collée à la couche de matériau absorbeur de liquide (115) avec un second adhésif (119).
